# EUROPEAN PATENT APPLICATION

(11) **EP 1 576 925 A1**
(43) Date of publication of application: **21.09.2005**
(21) Application number: 05005786.8
(22) Date of filing: 17.03.2005
(51) Int. Cl.: A61B 8/00, A61B 5/00

(54) **System and method for providing ultrasound images of a target object through a wireless communication network**

(30) Priority: 18.03.2004 KR 2004018227
(71) Applicant: MEDISON CO., LTD., Kangwon-do 250-870 (KR)
(72) Inventor: Kim, Sung Nam, Gunpo-si Gyeonggi-do 435-749 (KR)
(74) Representative: Schmid, Wolfgang

(57) **Abstract**

The present invention relates to a system for providing an ultrasound image of a target object, including: ultrasound diagnostic equipment for acquiring ultrasound data from the target object; an image converter for converting ultrasound data into mobile ultrasound data having a data format, which is suitable for transmission through a wireless communication network; and a mobile server for transmitting the mobile ultrasound data through the wireless communication network.

## Description

The present invention generally relates to a system for providing an ultrasound image, and more particularly to a system and a method for providing 2D or 3D ultrasound images of a target object through a wireless communication network.

Various technologies for acquiring an ultrasound image of a fetus have been researched by using ultrasound diagnostic equipment. Also, there are various efforts for managing the acquired ultrasound image of the fetus in order to provide the ultrasound image of the fetus to a pregnant woman and/or her family. Research of the ultrasound diagnostic equipment has been focused into a technology for storing and converting ultrasound data associated with the acquired ultrasound image of the fetus. Recently, a service for providing the ultrasound image of the fetus, which is acquired from the ultrasound diagnostic equipment, to the pregnant woman and/or her family is limitedly performed through a wire communication network. When the above service is performed, a computer connected to the wire communication network set up at a predetermined place should be used. Therefore, there is a problem in that it is extremely inconvenient to see the ultrasound image of the fetus since the place for seeing the ultrasound image is limited and predetermined.

It is an objective of the present invention to provide to a system and a method for providing 2D or 3D ultrasound images of a target object through a wireless communication network.

In accordance with an aspect of the present invention, there is provided a system for providing an ultrasound image of a target object, including: ultrasound diagnostic equipment for acquiring ultrasound data from the target object; a converter for converting ultrasound data into mobile ultrasound data having a data format, which is suitable for transmission through a wireless communication network; and a mobile server for transmitting the mobile ultrasound data through the wireless communication network.

In accordance with another aspect of the present invention, there is provided a method for providing an ultrasound image of a target object, including the steps of: a) acquiring ultrasound data from the target object; b) converting the ultrasound data into mobile ultrasound data having a data format, which is suitable for transmission through a wireless communication network; and c) transmitting the mobile ultrasound data through the wireless communication network.

In accordance with yet another aspect of the present invention, there is provided a method for providing an ultrasound image of a fetus, including the steps of: a) acquiring ultrasound data of fetus; b) storing the ultrasound data in a first storing unit, wherein the ultrasound data includes data associated with 2-dimensional or 3-dimensional ultrasound images of the fetus, medical information of the pregnant woman and the fetus and a heart beating sound; c) converting the ultrasound data of the fetus into mobile ultrasound data having a data format, which is suitable for transmission through a wireless communication network, wherein the mobile ultrasound data includes data associated with mobile ultrasound images of the fetus, mobile medical information of the pregnant woman and the fetus and a mobile heart beating sound; d) storing the mobile ultrasound data in a second storing unit; e) transmitting the mobile ultrasound data to a mobile device; and f) displaying the ultrasound image on the mobile device based on the mobile ultrasound data.

The above and other objects and features of the present invention will become apparent from the following description of preferred embodiments given in conjunction with the accompanying drawings, in which:
Fig. 1 is a block diagram showing a system for providing an ultrasound image of a fetus through a wireless communication network in accordance with the present invention;
Fig. 2 is a flow chart for generating the mobile ultrasound image data of the fetus in accordance with the present invention; and
Fig. 3 is a flow chart showing a process for transmitting the mobile ultrasound data of the fetus to a mobile device in accordance with the present invention.

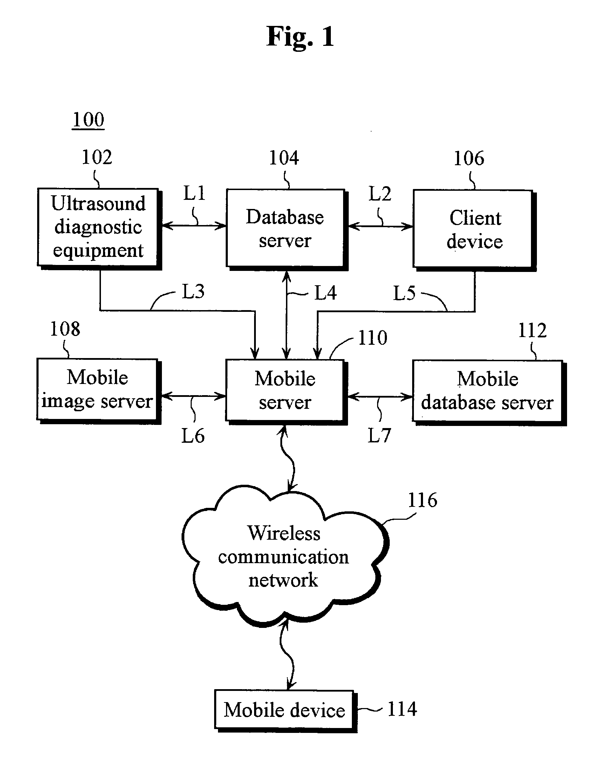

Fig. 1 is a block diagram showing a system for providing an ultrasound image of a fetus through a wireless communication network in accordance with the present invention.

Referring to Fig. 1, the system 100 of the present invention includes an ultrasound diagnostic equipment 102, a database server 104, a client device 106, a mobile image sever 108, a mobile server 110, a mobile database server 112 and a mobile device 114. The mobile server 110 and the mobile device 114 communicate with each other through a wireless communication network 116. Although ultrasound diagnostic equipment 102 and database server 104 are separately configured in Fig. 1, it is apparent to a person skilled in the art that data server 104 can be built in the ultrasound diagnostic equipment 102. Even if it is exemplarily described that mobile image server 108, mobile server 110 and mobile database server 112 are separately configured in Fig. 1 in accordance with the present invention, it is also apparent to a person skilled in the art that mobile image server 108 and mobile database server 112 can be configured within mobile server 110.

Ultrasound diagnostic equipment 102, which is a conventional ultrasound image device, acquires ultrasound data associated with an ultrasound image (i.e., a 2-dimensional (2D) or 3-dimensional (3D) ultrasound image) of a fetus from a pregnant woman. Ultrasound diagnostic equipment 102 transmits the acquired ultras data to database server 104 through a communication line L1. Thereafter, the ultrasound data are stored in one of the databases managed by the database server 104. An operation of database server 104 will be described later in detail. Ultrasound diagnostic equipment 102 may directly transmit the acquired ultrasound data, which is associated with the ultrasound image of the fetus, to the mobile server 110 through a communication line L3. The ultrasound data of the fetus includes data associated with not only ultrasound image but also medical information of the pregnant woman and the fetus and heartbeat sound of the fetus (i.e., Doppler sound).

Client device 104 includes a computing device such as a computer, a personal data assistant (PDA), a web pad, a mobile phone or the like. Client device 106 is connected to database server 104 through a communication line L2. Client device 106 retrieves the ultrasound data associated with the fetus stored in a plurality of databases managed by database server 104. The retrieved ultrasound data are stored in a storage space provided in client device 106. In order to transmit the retrieved or stored data associated with the fetus to mobile device 114, client device 106 transmits the data to mobile server through a communication line L5.

As mentioned above, the database server 104 includes a plurality of databases for storing the ultrasound data of the fetus. Each database stores the ultrasound data of the fetus transmitted from the ultrasound diagnostic device 102. The ultrasound data stored in the plurality of databases includes image data associated with the 2D or 3D ultrasound images of the fetus. Database server 104 retrieves the ultrasound data associated with ultrasound images of the fetus in response to requests from mobile server 110 and client device 106. It then transmits the retrieved ultrasound data of the fetus to the mobile server 110 and client device 106 through communication lines L2 and L4.

Mobile server 110 transmits the ultrasound data of the fetus, which is to be transmitted in response to the request from mobile device 114 to mobile image server 108 through a communication line L6. Mobile server 110 receives mobile ultrasound data, which are converted from the ultrasound data of the fetus in mobile image server 108. Thereafter, mobile server 110 transmits the mobile ultrasound data to mobile database server 112 so as to be stored in mobile database server 112. Mobile server 110 transmits the mobile ultrasound data to mobile device 114 in response to the request from mobile device 114. Before retrieving the ultrasound data, mobile server 110 may perform a user certification procedure for a user of mobile device 114 through a predetermined user certification algorithm. Since the user certification procedure is already well known, a detailed description thereof will be omitted herein.

The mobile image server 108 converts the ultrasound data transmitted from mobile server 110 into the mobile ultrasound data having a data format which is suitable for being transmitted to mobile device 114. The mobile ultrasound data includes data associated with mobile ultrasound images of the fetus, mobile medical information of the pregnant woman and the fetus and a mobile heart beating sound. Mobile image server 108, as mentioned above, transmits the mobile ultrasound data to mobile database server through mobile server 110 for storing the mobile ultrasound data in the mobile database server 112. When the user requests to edit the ultrasound image, the mobile ultrasound image can be edited in mobile image server 112 by superposing various clip art images or inserting letters to a selected mobile ultrasound image in response to an image edit request, which is inputted through mobile server 110, from mobile device 114.

Mobile database server 112 manages a plurality of databases. Each database stores mobile ultrasound data received from mobile server 110 to mobile image server 108. Mobile database server 112 retrieves mobile ultrasound data stored in the plurality of databases in response to a request from mobile server 110. It then transmits the retrieved mobile ultrasound data to mobile server 110. Also, each database included in mobile database server 112 stores lists associated with the mobile ultrasound data, respectively. When mobile data server transmits the mobile ultrasound data to mobile server 110, the list associated with mobile ultrasound data is also provided to mobile device 114 through mobile server 110.

Mobile device 114 is a mobile computing device such as a mobile phone to be accessed in mobile server 110 through wireless communication network 116. Mobile device 114 includes an instruction input unit and a display unit (not shown). The instruction input unit inputs instructions for requesting mobile ultrasound data transmission to mobile server 110. The display unit receives the mobile ultrasound data from mobile server 110 and displays the mobile ultrasound image based on the received mobile ultrasound data. Also, mobile device 114 further includes an audio playing unit (not shown) for playing the heart beating sound based on sound data of the fetus included in the mobile ultrasound data.

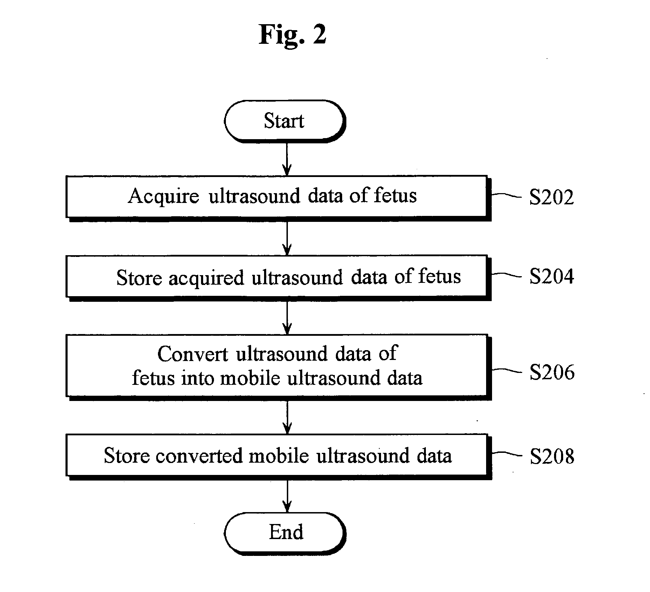

Fig. 2 is a flow chart for generating the mobile ultrasound image data of the fetus in accordance with the present invention. Hereinafter, a process for generating the mobile ultrasound data of the fetus will be described by referring to Figs. 1 and 2.

Ultrasound diagnostic equipment 102 acquires the ultrasound data of the fetus from the pregnant woman at step S202. Database server 104 stores the ultrasound data acquired from ultrasound diagnostic equipment 102 at step S204. As described above, the ultrasound data includes data associated with not only 2D or 3D ultrasound image of the fetus but also medical information of the pregnant woman and the fetus and the heart beating sound of the fetus.

Mobile server 110 transmits the ultrasound data of the fetus, which is outputted from database server 104, to mobile image server 108 through communication line L6 at step S206. Thereafter, mobile image server 108 converts the transmitted ultrasound data of the fetus into the mobile ultrasound data, which have a data format suitable for being transmitted to mobile device 114 through wireless communication network 116, by using a predetermined image conversion algorithm. Next, mobile server 110 transmits the mobile ultrasound data transmitted from mobile image server 108 through communication line L7, thereby storing the mobile ultrasound data to mobile database server 112.

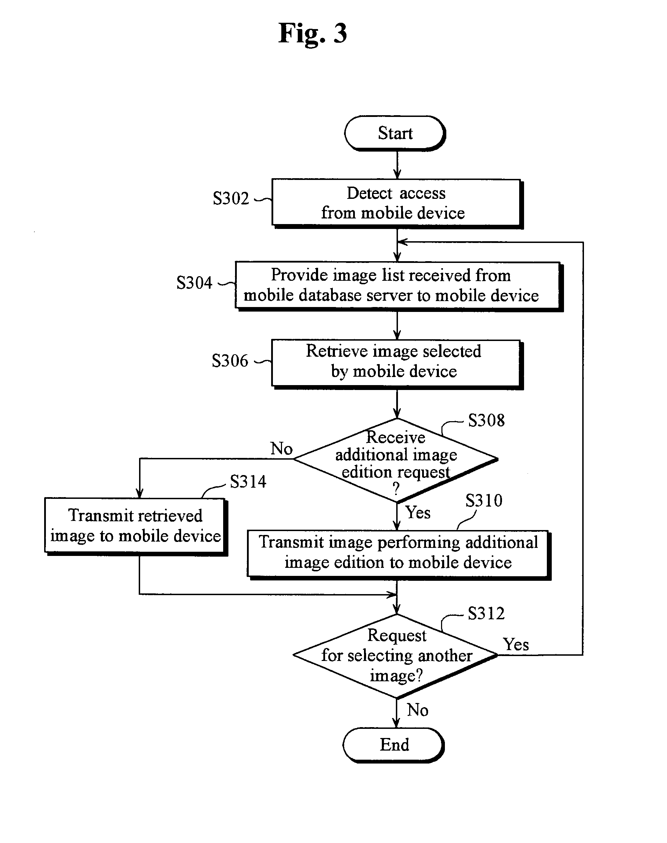

Fig. 3 is a flow chart showing a process for transmitting the mobile ultrasound data of the fetus to mobile device 114 in accordance with the present invention. Hereinafter, the process for transmitting the mobile ultrasound data to mobile device 114 will be described in detail by referring to Figs. 1 to 3.

Mobile server 110 detects whether mobile device 114 is accessed through wireless communication network 116 at step S302. At this step S302, mobile server 110 performs a user certification process for the user of mobile device 114 by using a conventional certification algorithm.

Next, the mobile server 110 transmits the list associated with mobile ultrasound data received from mobile database server 112 to mobile device 114 through wireless communication network 116 at step S304. Mobile device 114 transmits an image request signal for requesting an image selected by the user to mobile server 110. Mobile server 110 retrieves the selected image from mobile database server 112 in response to the image request signal at step S306.

The mobile server 110 checks whether an additional image edition request is received from mobile device 114 at step S308. If it is determined that the additional image edition request is not received, the process proceeds to step S314. Mobile server 110 transmits the mobile ultrasound data associated with the image retrieved at step S306 to mobile device through wireless communication network 116. This is so that the mobile device 114 displays the mobile ultrasound image based on the received mobile ultrasound data.

Meanwhile, if it is determined that the additional image edition request is received at step S308, the process proceeds to step S310. In order to perform the requested additional image edition at step S310, the mobile ultrasound data associated with the mobile ultrasound image retrieved at step S310 are transmitted to mobile image server 108. After editing the mobile ultrasound image received from mobile image server 108, mobile image server 108 transmits the edited mobile ultrasound data to mobile server 110. The additional image edition, as mentioned above, means inserting the clip art image or letters on the mobile ultrasound image. The mobile ultrasound data including data, which the additional image edition has been performed, are transmitted to mobile device 114 through wireless communication network 116. Mobile device 114 displays the mobile ultrasound image and plays the heart beating sound based on the received mobile ultrasound data from mobile server 110.

Mobile server 110 checks whether the image selection request signal for selecting another images is received from mobile device 114 at step S312. If it is determined that the image selection request signal is received, the process returns to step S304 for performing above process again. On the other hand, if it is determined that the image selection request signal is not received, the process is completed in accordance with the present invention.

As described in above, since the mobile ultrasound data are transmitted to the mobile device such as the mobile phone by converting the data format of the 2D or 3D ultrasound image data to be displayed on the mobile device, there is an effect that the pregnant woman or her family can see the ultrasound image of fetus transmitted through wireless communication network regardless of restriction of space and time. Also, since the clip art image or letters can be inserted in the ultrasound image of the fetus in accordance with the present invention, there is an effect that the ultrasound image can be edited to pregnant woman's taste.

While the present invention has been described and illustrated with respect to a preferred embodiment of the invention, it will be apparent to those skilled in the art that variations and modifications are possible without deviating from the broad principles and teachings of the present invention which should be limited solely by the scope of the claims appended hereto.

## Claims

1. A system for providing an ultrasound image of a target object, comprising:
means for acquiring ultrasound data from the target object;
means for converting the ultrasound data into mobile ultrasound data having a data format, which is suitable for transmission through a wireless communication network; and
means for transmitting the mobile ultrasound data through the wireless communication network.

2. The system as recited in claim 1, wherein the target object is a fetus.

3. The system as recited in claim 2, wherein the ultrasound data includes data associated with 2-dimensional ultrasound images of the fetus, 3-dimensional ultrasound images of the fetus, medical information of a pregnant woman and the fetus and a heart beating sound.

4. The system as recited in claim 3, further comprising a mobile device for displaying a mobile ultrasound image and playing a heat beating sound based on the mobile ultrasound data.

5. The system as recited in claim 4, further comprising:
a first database for storing the ultrasound data of the fetus; and
a second database for storing the mobile ultrasound data converted from the ultrasound data.

6. The system as recited in claim 5, further comprising means for editing a mobile ultrasound image based on the mobile ultrasound data by inserting clip art images or letters.

7. A method for providing an ultrasound image of a target object, comprising the steps of:
a) acquiring ultrasound data from the target object;
b) converting the ultrasound data into mobile ultrasound data having a data format, which is suitable for transmission through a wireless communication network; and
c) transmitting the mobile ultrasound data through the wireless communication network.

8. The system as recited in claim 7, wherein the target object is a fetus.

9. The system as recited in claim 8, wherein the ultrasound data includes data associated with 2-dimensional ultrasound images of the fetus, 3-dimensional ultrasound images of the fetus, medical information of a pregnant woman and the fetus and a heart beating sound.

10. The method as recited in claim 9, further comprising the steps of:
d) storing the ultrasound data of the fetus; and
e) storing the mobile ultrasound data converted from the ultrasound data.

11. A method for providing an ultrasound image of a fetus, comprising the steps of:
a) acquiring ultrasound data of fetus;
b) storing the ultrasound data in a first storing unit, wherein the ultrasound data includes data associated with 2-dimensional images of the fetus, 3-dimensional ultrasound images of the fetus, medical information of the pregnant woman and the fetus and a heart beating sound;
c) converting the ultrasound data of the fetus into mobile ultrasound data having a data format, which is suitable for transmission through a wireless communication network, wherein the mobile ultrasound data includes data associated with mobile ultrasound images of the fetus, mobile medical information of the pregnant woman and the fetus and a mobile heart beating sound;
d) storing the mobile ultrasound data in a second storing unit;
e) transmitting the mobile ultrasound data to a mobile device; and
f) displaying the ultrasound image on the mobile device based on the mobile ultrasound data.

12. The method as recited in claim 11, wherein the second storing unit includes a list associated with the mobile ultrasound data.

13. The method as recited in claim 12, wherein the step e) includes the steps of:
e1) transmitting the list to the mobile device;
e2) selecting one of mobile ultrasound images in the mobile device;
e3) retrieving a selected mobile ultrasound image in the second storing unit; and
e4) transmitting a retrieved mobile ultrasound image to the mobile device.

14. The method as recited in claim 13, wherein the step e4) includes the step of e41) checking whether a additional image edition request for the retrieved mobile ultrasound image is received from the mobile device.

15. The method as recited in claim 14, wherein, if it is determined that the additional image edition request is received at step e41), the retrieved mobile ultrasound image is edited by inserting predetermined clip art images or letters.

16. The method as recited in claim 14, wherein, if it is determined that the additional image edition request is not received at step e41), the retrieved mobile ultrasound image is transmitted to the mobile device.
